# EUROPEAN PATENT APPLICATION

(11) **EP 1 607 040 A2**
(43) Date of publication of application: **21.12.2005**
(21) Application number: 05012786.9
(22) Date of filing: 14.06.2005
(51) Int. Cl.: A61B 5/02

(54) **Electronic blood pressure monitor**

(30) Priority: 17.06.2004 JP 2004179050
(71) Applicant: Omron Healthcare Co., Ltd., Kyoto 615-0084 (JP)
(72) Inventor: Inoue, Tomonori, Yamanoshita-cho Ukyo-ku Kyoto 615-0084 (JP); Ishida, Tomoya, Yamanoshita-cho Ukyo-ku Kyoto 615-0084 (JP); Nakanishi, Hiroya, Yamanoshita-cho Ukyo-ku Kyoto 615-0084 (JP)
(74) Representative: Kilian, Helmut

(57) **Abstract**

An electronic blood pressure monitor has a main body (10) provided with an arm insertion portion (11) having a throughhole and provided with a cylindrical cufF, that is provided with an elbow rest (20) receiving an elbow of an arm inserted through and thus placed outside the arm insertion portion (11). The elbow rest (20) is rotatably supported at a lower portion of a rear side of the main body (10) by a support shaft (15) to be positionally changeable between elbow supporting and unused positions and at the unused position it is accommodated by the main body (10) upper than the support shaft (15). The elbow rest (20) includes a proximal support (20), a distal support (22) and an elbow support (23). When blood pressure is measured the elbow rest (20) is set to take the elbow supporting position to receive an elbow placed outside the arm insertion portion (11). An advantage of a home blood pressure monitor can be maintained and accurate blood pressure measurement can also be obtained.

## Description

### BACKGROUND OF THE INVENTION

### Field of the Invention

The present invention relates to electronic blood pressure monitors attached to a living body (an arm in particular) and thus employed to measure blood pressure.

### Description of the Background Art

Electronic blood pressure monitors that can automatically wrap a cuff around a subject can be divided into two types: one is medical blood pressure monitors introduced in hospitals and the like and the other is miniaturized and portable, home blood pressure monitors.

One medical blood pressure monitor is a blood pressure measurement apparatus including a body having a display unit displaying a measured value or the like, a cuff unit automatically wrapping a cuff around an arm inserted therethrough, and a printer printing out the measured value or the like. This type of blood pressure monitor is disclosed for example in Japanese Patent Laying-Open No. 2000-217794.

While medical blood pressure monitors are large in size and weight, as disclosed in Japanese Patent Laying-Open No. 2000-217794, home blood pressure monitors adopt an advantage of the medical blood pressure monitors, i.e., automatically wrapping a cuff, and an advantage of a portable blood pressure monitor wrapping a cuff manually, i.e., a miniature and light weight structure, to be enhanced in portability.

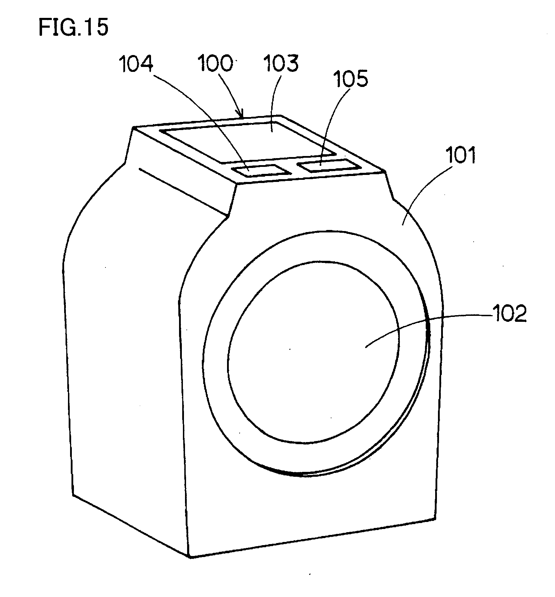

Such a home blood pressure monitor is provided in a form such as shown in Fig. 15. The figure shows a blood pressure monitor 100 including a main body 101 provided with an arm insertion portion 102 having a throughhole and provided with a cylindrical cuff (not shown). Main body 101 is also provided with a display unit 103, a measurement start button 104, a measurement stop button 105, and the like.

Such electronic blood pressure monitor 100 has a variety of improvements. For example, Japanese Patent Laying-Open No. 2003-038451 discloses a cuff apparatus having an air bag accommodated inner than a chassis cylindrically and receiving pressurized air to prevent a site to be measured from having blood passing therethrough, that includes a cushion member arranged internal to the air bag to keep the air bag expanding before the pressurized air is introduced thereinto.

Home blood pressure monitors including those having the cuff device disclosed in Japanese Patent Laying-Open No. 2003-038451 are required to be as small in size as possible and accordingly often have elbow rest, a cuff angle adjustment function and the like eliminated therefrom. As such, when a user inserts his/her arm into the cuff for measurement the user cannot determine how deep his/her arm should be inserted therethrough, and may well insert his/her arm to incorrect depth and/or take an incorrect posture, position or the like for measurement, resulting in incorrect blood pressure measurement. If such disadvantage of the home blood pressure monitor is overcome by introducing a medical blood pressure monitor's elbow rest, cuff angle adjustment function and the like, the home blood pressure monitor would be increased in size, which impairs the home blood pressure monitor's advantage and the home blood pressure monitor would be inconvenient for use.

### SUMMARY OF THE INVENTION

The present invention contemplates a blood pressure monitor that does not impair an advantage as a home blood pressure monitor and can still provide correct blood pressure measurement.

To achieve the above object the present electronic blood pressure monitor has a main body provided with an arm insertion portion having a throughhole and provided with a cylindrical cuff, that is provided with an elbow rest receiving an elbow of an arm inserted through and thus placed external to the arm insertion portion, the elbow rest being positionally changeable between an elbow supporting position and an unused position.

The present electronic blood pressure monitor in blood pressure measurement has the elbow rest at the elbow supporting position to receive an elbow placed outside the arm insertion portion, otherwise has the elbow rest at the unused position. The elbow rest that is positionally changeable can be out of the way in the unused position, and when it is set to have the elbow supporting position it allows an appropriate posture or position for measurement to be achieved. While a home blood pressure monitor's advantageous smallness in size can be maintained and accurate blood pressure measurement can also be obtained. Note that "positionally changeable" as referred to herein means that the elbow rest is configured such that when the electronic blood pressure monitor is placed for example on a table and the elbow rest is shifted from the unused position to the elbow supporting position the elbow rest will occupy a displaced, spatially different position, and it does not necessarily mean an arrangement limiting the elbow rest to be positionally changeable relative to the main body.

In the present electronic blood pressure monitor preferably the elbow rest is positionally changeable between the elbow supporting position and the unused position relative to the main body. In that case the elbow rest is better supported rotatably by a support shaft provided to the main body.

In the present electronic blood pressure monitor preferably the elbow rest is supported between the elbow supporting position and the unused position rotatably by the support shaft provided to the main body and at the unused position is accommodated by the main body upper than the support shaft. In that case, for measurement the elbow rest is rotated to the elbow supporting position, otherwise it is accommodated by the main body upper than the support shaft to take the unused position.

Furthermore in the present electronic blood pressure monitor preferably the elbow rest is supported between the elbow supporting position and the unused position rotatably by the support shaft provided to the main body and at the unused position is accommodated by the main body lower than the support shaft. In that case, when blood pressure is not measured the elbow rest is accommodated by the main body lower than the support shaft to take the unused position.

Furthermore in the present electronic blood pressure monitor preferably the elbow rest is rotatably attached by the support shaft provided to the main body so that the elbow rest at the unused position functions as a leg supporting the main body and at the elbow supporting position functions as a leg supporting the main body as well as an elbow rest. In that case, for blood pressure measurement the elbow rest is rotated to take the elbow supporting position allowing the elbow rest to function as a leg supporting the main body as well as an elbow rest, otherwise it is rotated to take the unused position allowing the elbow rest to function only as the leg supporting the main body.

In the present electronic blood pressure monitor preferably the elbow rest is slidably input to and output from and thus accommodated in the main body. In that case, for blood pressure measurement the elbow rest is slid out the main body to take the elbow supporting position, otherwise it is slid into the main body to take the unused position.

Furthermore in the present electronic blood pressure monitor preferably the elbow rest is detachably attachable to the main body.

Furthermore in the present electronic blood pressure monitor preferably the elbow rest is formed to be integral with the main body to function as a leg supporting the main body as well as an elbow rest when the elbow rest has the elbow supporting position. In that case preferably the elbow rest protrudes from the main body. Furthermore, in that case, for blood pressure measurement for example the main body is inclined to achieve the elbow supporting position allowing the elbow rest to function as a leg supporting the main body as well as an elbow rest, otherwise the main body is returned to its initial position to allow the elbow rest to take the unused position.

Furthermore in the present electronic blood pressure monitor preferably the elbow rest has a geometry supporting an inclined position of the main body allowing the arm insertion portion to be obliquely downward from a proximal side toward a distal side when the elbow rest is positionally changed to take the elbow supporting position. In that case, in blood pressure measurement when the elbow rest is set to take the elbow supporting position the main body inclines and accordingly the arm insertion portion inclines from the proximal side toward the distal side obliquely downward and when an elbow protruding from the arm insertion portion is placed on the elbow rest the arm can be held through the arm insertion portion naturally.

Another electronic blood pressure monitor has a main body provided with an arm insertion portion having a throughhole and provided with a cylindrical cuff, characterized in that an elbow rest is detachably attached to the main body to receive an elbow of an arm inserted through and thus placed outside the arm insertion portion.

The present electronic blood pressure monitor in blood pressure measurement has the elbow rest attached to the main body to take an elbow supporting position, otherwise has the elbow rest detached from the main body to take an unused position.

Furthermore another electronic blood pressure monitor has a main body provided with an arm insertion portion having a throughhole and provided with a cylindrical cuff, characterized in that an elbow rest receiving an elbow of an arm inserted through and thus placed outside the arm insertion portion protrudes from the main body.

Desirably the above described electronic blood pressure monitors have their respective elbow rests each formed of a proximal support supporting a proximal portion of an arm with respect to an elbow and a distal support bent and thus directly adjacent to the proximal support to support a distal portion of the arm with respect to the elbow, and the proximal support and the distal support form an inflection serving as an elbow support. Thus when the elbow rest is set to take the elbow supporting position the elbow can be placed thereon to take an ergonomically natural position.

In accordance with the present invention there can be provided an elbow rest that can guide an inserted arm to correct depth. Blood pressure can be measured at a correct site for measurement and correct blood pressure measurement can thus be obtained. In addition, when blood pressure is not measured, the elbow rest can be out of the way and the blood pressure monitor can thus require smaller space, and furthermore provide a blood pressure monitor serving as a home blood pressure monitor requiring only a small space.

Furthermore in blood pressure measurement when the elbow rest is set to take the elbow supporting position the arm insertion portion can be inclined from a proximal side to a distal side obliquely downward. An elbow placed outside the main body's arm insertion portion can be rested on the elbow rest naturally so that a more natural posture or position for measurement can be achieved and more accurate blood pressure measurement can thus be provided.

Thus a home blood pressure monitor's advantages (small in size, light-weight and portable) can be intact and high precision blood pressure measurement can also be obtained.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic cross section of the present electronic blood pressure monitor in a first embodiment.
Fig. 2 is a top plan view of an elbow rest of the present electronic blood pressure monitor in the first embodiment.
Fig. 3 is a bottom plan view of the elbow rest of the present electronic blood pressure monitor in the first embodiment.
Figs. 4, 5 and 6 are schematic cross sections of the present electronic blood pressure monitor in second, third and fourth embodiments, respectively.
Figs. 7 and 8 are schematic cross sections of the present electronic blood pressure monitor in a fifth embodiment with an elbow rest accommodated and drawn out, respectively.
Fig. 9 is a schematic cross section of the present electronic blood pressure monitor in a sixth embodiment with an elbow rest unused.
Fig. 10 is a schematic cross section of the present electronic blood pressure monitor in the sixth embodiment with the elbow rest at an elbow supporting position.
Fig. 11 is a schematic cross section of the present electronic blood pressure monitor in a seventh embodiment with an elbow rest unused.
Fig. 12 is a schematic cross section of the present electronic blood pressure monitor in the seventh embodiment with the elbow rest at an elbow supporting position.
Fig. 13 is a schematic cross section of the present electronic blood pressure monitor in an eighth embodiment with an elbow rest attached to the main body.
Fig. 14 is an external perspective view of one example of the main body of the electronic blood pressure monitor in the eighth embodiment.
Fig. 15 is an external perspective view of one example of a conventional electronic blood pressure monitor.

### DESCRIPTION OF THE PREFERRED EMBODIMENTS

Hereinafter with reference to embodiments the present invention will be described more specifically.

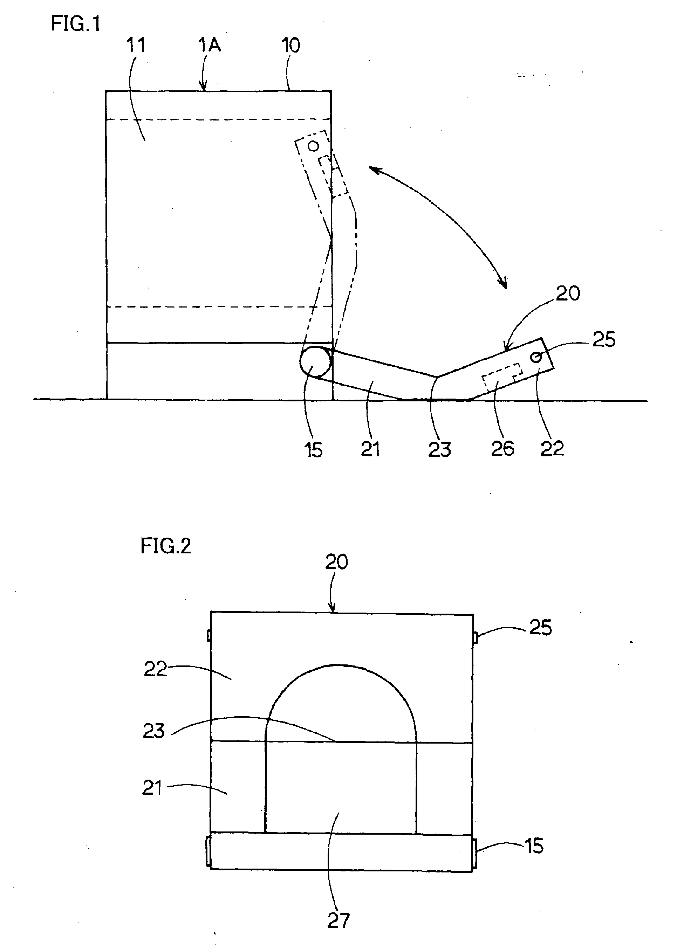

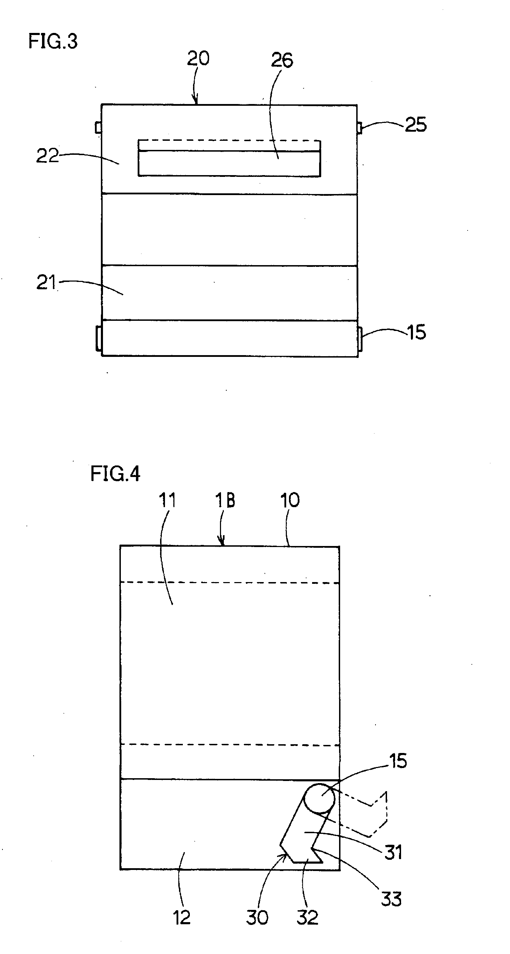

A first embodiment provides an electronic blood pressure monitor 1A, as shown in Fig. 1 in a schematic cross section, with an elbow rest, as shown in Figs. 2 and 3 in a top plan and bottom plan views, respectively.

Electronic blood pressure monitor 1A includes a main body 10 which is provided with an arm insertion portion 11 having a throughhole and provided with a cylindrical cuff (not shown) and has an elbow rest 20 attached thereto to support an elbow of an arm inserted through and thus placed external to arm insertion portion 11. Elbow rest 20 is adapted to be positionally changeable between an elbow supporting position (indicated by a solid line) and an unused position (indicated by a chained line). Although not shown in the figure, main body 10 is equipped with a circuit board, an air pump, a piping system, a console and the like.

Elbow rest 20 is rotatably supported by a support shaft 15, provided at a lower portion of a rear side of main body 10, between the elbow supporting position and the unused position. At the unused position, elbow rest 20 is accommodated by main body 10 upper than support shaft 15. Elbow rest 20 is configured of a proximal support 21 supporting a proximal portion of an arm with respect to the elbow (i.e., a portion of the arm closer to the shoulder with respect to the elbow) and a distal support 22 bent from and thus directly adjacent to proximal support 21 for supporting a distal portion of the arm with respect to the elbow (i.e., a portion of the arm closer to the hand) and between proximal support 21 and distal support 22 there is an inflection serving as an elbow support 23. As is apparent from Fig. 1, elbow rest 20 is formed in a geometry that allows an elbow to rest thereon ergonomically naturally.

Furthermore, elbow rest 20 has at distal support 22 a protrusion 25 for holding an accommodated position (or the unused position) and a handle 26 used to pull down the elbow rest from the accommodated position, and is also provided in an elbow supporting side with a recess 27 in the form of the letter U to prevent an arm including an elbow from positional displacement. Main body 10 at a portion corresponding to protrusion 25 of elbow rest 20 is provided with a recess (not shown) engaging with protrusion 25, and engaging protrusion 25 with the recess holds elbow rest 20 at a position allowing the elbow rest to be accommodated by main body 10. Holding handle 26 and thus pulling down elbow rest 20 from this accommodated position allows the elbow rest to have the elbow supporting position indicated by the solid line.

In Fig. 1, electronic blood pressure monitor 1A in blood pressure measurement has elbow rest 20 pulled down, as indicated by the solid line, to have the elbow supporting position and a user inserts his/her arm into main body 10 through arm insertion portion 11. For the sake of illustration, the arm is inserted from the left hand to the right hand as seen in the plane of the figure. In doing so, the arm is inserted such that the elbow rests on elbow rest 20 at elbow support 23, and the elbow is thus rested on elbow rest 20. Thus the user can clearly understand how deep he/she should insert his/her arm The user can thus insert his/her arm to a correct depth (or position) and correct blood pressure measurement can thus be obtained.

When blood pressure is not measured, elbow rest 20 has protrusion 25 engaged with the recess of main body 10 to bring elbow rest 20 to the unused position. Thus when blood pressure is not measured elbow rest 20 can be out of the way and thus occupy reduced space.

A second embodiment provides an electronic blood pressure monitor 1B as shown in Fig. 4 in a schematic cross section. Note that components identical to those of electronic blood pressure monitor 1A are identically denoted. Electronic blood pressure monitor 1B has an elbow rest 30 accommodated in main body 10 lower than support shaft 15 when it has an unused position. More specifically, elbow rest 30 at the unused position (indicated by a solid line) is accommodated in a housing 12 provided at a lower portion of main body 10, and at an elbow supporting position (indicated by a chained line) is drawn out of housing 12. Elbow rest 30 is configured of a proximal support 31, a distal support 32 and an elbow support 33, as has been described previously, except that distal support 32 is set to be shorter than in the first embodiment. More specifically, distal support 32 may not have a length that can substantially support an arm's distal portion (a portion closer to the hand). Furthermore, elbow rest 30 is adapted so that its elbow supporting and unused positions are each locked for example by engaging a protrusion and a recess.

When electronic blood pressure monitor 1B is used to measure blood pressure elbow rest 30 is drawn out, as indicated by the chained line, to have the elbow supporting position, otherwise elbow rest 30 is accommodated in housing 12, as indicated by the solid line, to have the unused position. As a matter of course, electronic blood pressure monitor 1B can provide a function and effect equivalent to that of electronic blood pressure monitor 1A.

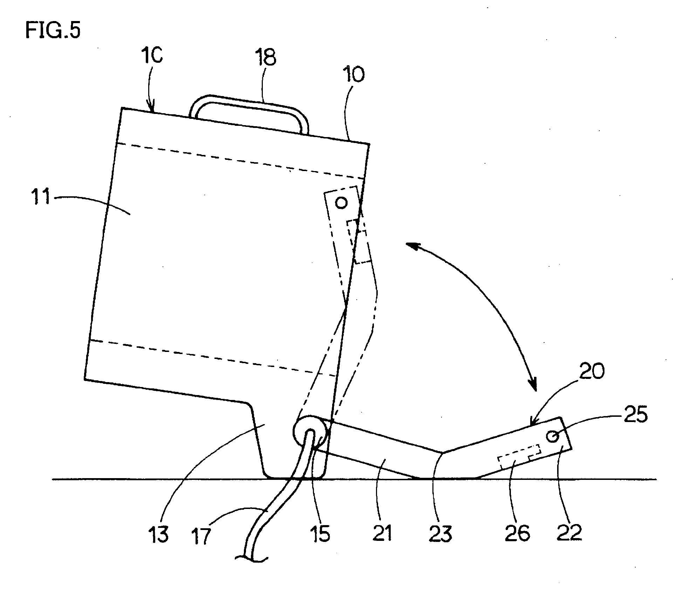

A third embodiment provides an electronic blood pressure monitor 1C shown in Fig. 5 in a schematic cross section. For electronic blood pressure monitor 1C, main body 10 has a lower (or bottom) portion partially serving as a leg 13 supporting main body 10 in blood pressure measurement shown in Fig. 5. Elbow rest 20 in measurement also functions as a leg supporting main body 10. Furthermore, leg 13 has a geometry supporting that inclined position of main body 10 at which arm insertion portion 11 inclines from a proximal side toward a distal side obliquely downward in blood pressure measurement.

For electronic blood pressure monitor 1C, main body 10 only has a cuff function. Other functions (the circuit board, the air pump, the console and the like) are provided at a separate main body (not shown) connected via an air tube 17. Furthermore, main body 10 has a top portion provided with a handle 18 enhancing main body 10 in portability. Elbow rest 20 is identical to that of electronic blood pressure monitor1A.

When electronic blood pressure monitor 1C is used to measure blood pressure and elbow rest 20 is accordingly set to take the elbow supporting position, main body 10 inclines and accordingly arm insertion portion 11 inclines from the proximal side toward the distal side obliquely downward. This can help a user to insert his/her arm through arm insertion portion 11 and, with the elbow outside arm insertion portion 11 rested on elbow rest 20, arm insertion portion 11 adapts to an inclination of the arm (the upper arm in particular). Thus in measurement the user can take a further more natural posture or position and more accurate blood pressure measurement can thus be obtained.

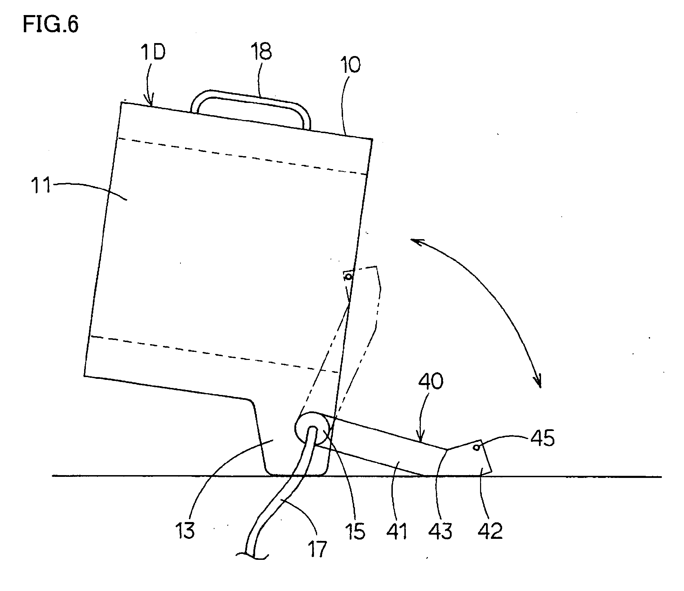

A fourth embodiment provides an electronic blood pressure monitor 1D shown in Fig. 6 in a schematic cross section. Electronic blood pressure monitor 1D is an exemplary variation of electronic blood pressure monitor 1C and has an elbow rest 40 similarly configured of a proximal support 41, a distal support 42 and an elbow support 43, except that distal support 42 is set to be shorter, similarly as has been described in the second embodiment. As a matter of course, elbow rest 40 has a protrusion 45 for holding elbow rest 40 at an accommodated position.

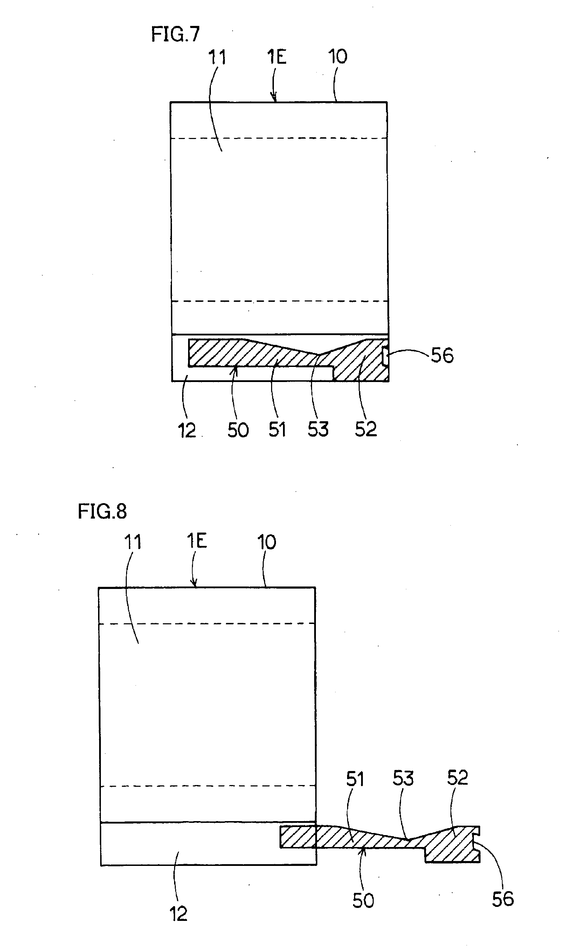

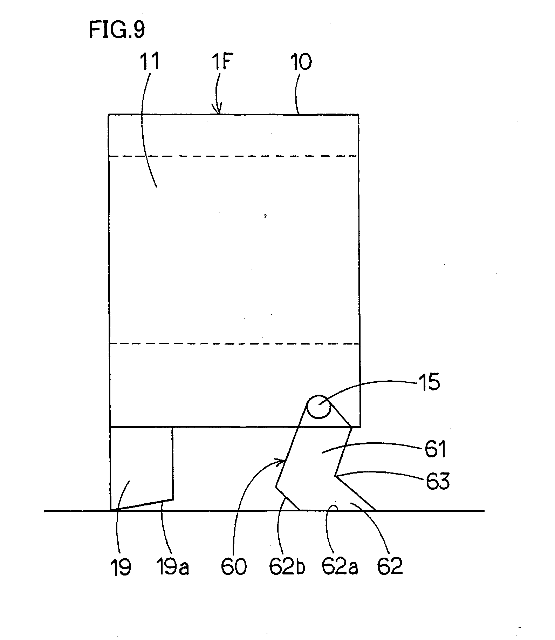

A fifth embodiment provides an electronic blood pressure monitor 1E as shown in Figs. 7 and 8 in schematic cross sections with an elbow rest accommodated and drawn out, respectively. Electronic blood pressure monitor 1E has an elbow rest 50 slidably input and output and thus accommodated in housing 12 provided at a lower portion of main body 10. Elbow rest 50 is configured of a proximal support 51, a distal support 52 and an elbow support 53, and distal support 52 has an end provided with a handle 56. When elbow rest 50 takes the Fig. 8 elbow supporting position, distal support 52 also functions as a leg supporting elbow rest 50.

When electronic blood pressure monitor 1E is used to measure blood pressure elbow rest 50 is drawn out, as indicated in Fig. 8, to have the elbow supporting position, otherwise elbow rest 50 is accommodated in housing 12, as indicated in Fig. 7, to have the unused position.

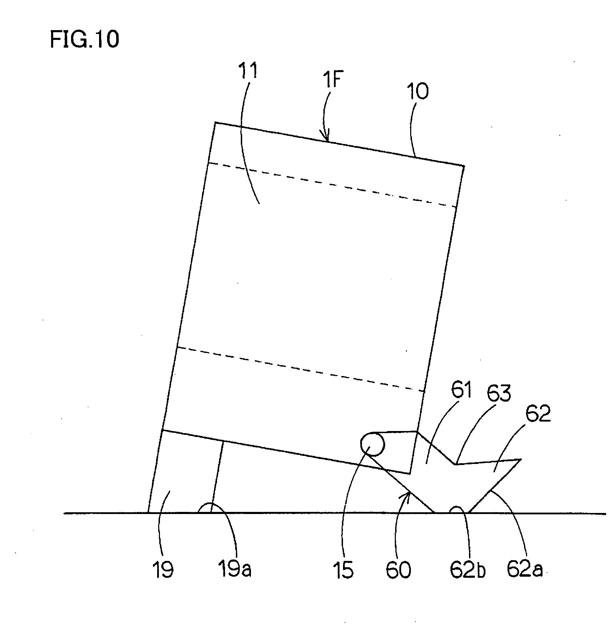

A sixth embodiment provides an electronic blood pressure monitor 1F shown in Figs. 9 and 10 in schematic cross sections with an elbow rest at unused and elbow supporting positions; respectively. For electronic blood pressure monitor 1F, main body 10 has a bottom portion provided with a leg 19 and an elbow rest 60 also serving as a leg. Leg 19 has an inclined bottom surface 19a. When elbow rest 60 takes the unused position leg 19 has one side in contact with a surface. When elbow rest 60 takes the elbow supporting position, leg 19 has bottom surface 19a in contact with the surface. Elbow rest 60 is configured of a proximal support 61, a distal support 62 and an elbow support 63, and at the unused position distal support 62 has an end surface 62a in contact with the surface and at the elbow supporting position distal support 62 has an end surface 62b in contact with the surface. In other words, when elbow rest 60 has the unused position it only functions as a leg and when elbow rest 60 has the elbow supporting position it serves as a leg as well as an elbow rest.

When electronic blood pressure monitor 1F has elbow rest 60 at the unused position, as shown in Fig. 9, main body 10 is supported by leg 19 and elbow rest 60. In blood pressure measurement when elbow rest 60 is set to have the elbow supporting position, as shown in Fig. 10, main body 10 is also supported by leg 19 and elbow rest 60. It should be noted, however, that as well as electronic blood pressure monitors 1C and 1D, electronic blood pressure monitor 1F similar inclines and accordingly arm insertion portion 11 inclines from a proximal side toward a distal side obliquely downward. This can help the user to insert his/her arm through arm insertion portion 11 and, with the elbow outside arm insertion portion 11 rested on elbow rest 60 at elbow support 63, arm insertion portion 11 adapts to an inclination of the arm (the upper arm in particular). Thus in measurement the user can take a further more natural posture or position and more accurate blood pressure measurement can thus be obtained.

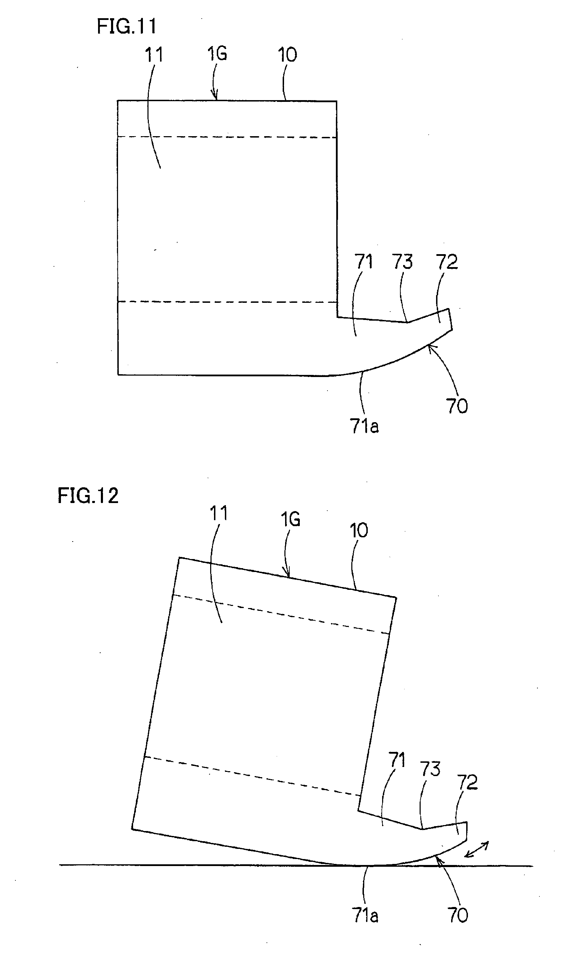

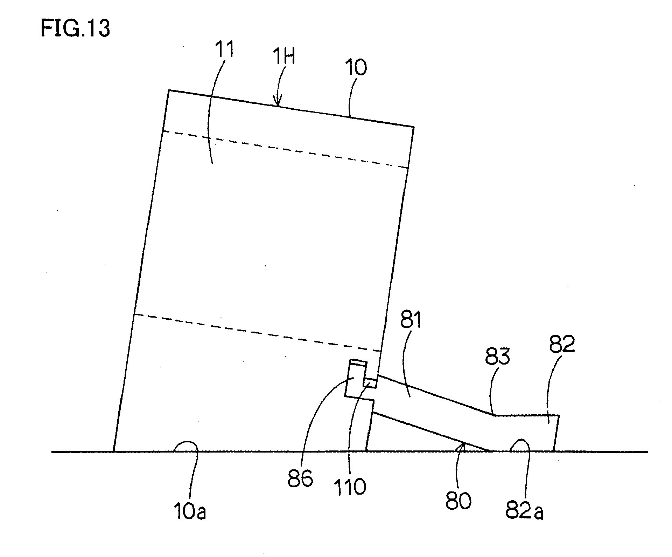

A seventh embodiment provides an electronic blood pressure monitor 1G shown in Figs. 11 and 12 in schematic cross sections with an elbow rest at unused and elbow supporting positions, respectively. Electronic blood pressure monitor 1G has an elbow rest 70 formed to be integral with and thus protruding from a lower portion of a rear side of main body 10. Elbow rest 70 is configured of a proximal support 71, a distal support 72 and an elbow support 73 with their bottom surface 71a generally curved. While elbow rest 70 at the unused position simply protrudes from the rear side of main body 10, at the elbow supporting position, inclining main body 10 brings curved bottom surface 71 a into contact with a surface to support main body 10 in an inclined position. In other words, elbow rest 70 at the elbow supporting position serves as a leg as well as an elbow rest.

When electronic blood pressure monitor 1G is used to measure blood pressure the elbow rest 70 curved bottom surface 71a can support main body 10 to allow main body 10 or arm insertion portion 11 to be inclined at any angle depending on the user. This is done because different users have their arms different in thickness, length and the like depending on their physiques, and when a user inserts his/her arm into main body 10 through arm insertion portion 11 it is better that arm insertion portion 11 can be inclined at an angle that can be adjusted, as desired, to correspond to the user, rather than fixed. As a matter of course, elbow rest 70 may have an inclined bottom surface as provided for elbow rest 60 of electronic blood pressure monitor 1F (Figs. 9 and 10).

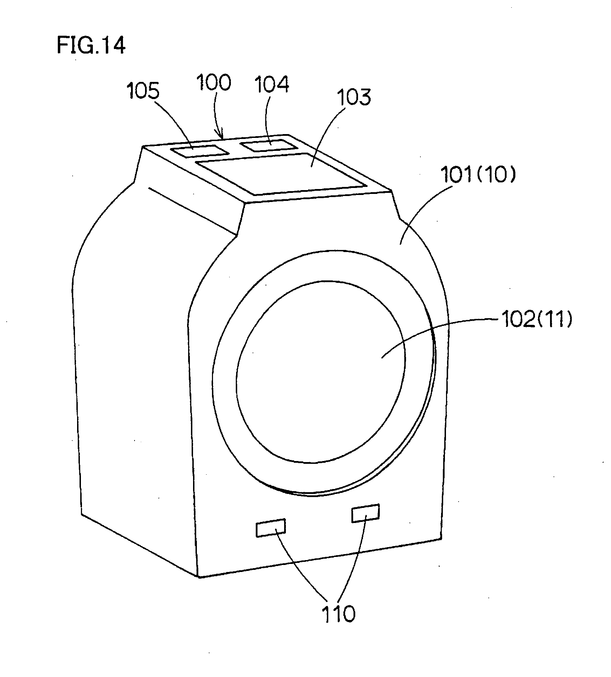

An eighth embodiment provides an electronic blood pressure monitor 1H shown in Fig. 13 in a schematic cross section. For electronic blood pressure monitor 1H, an elbow rest 80 is detachably attached to main body 10. Elbow rest 80 is configured of a proximal support 81, a distal support 82 and an elbow support 83, and distal support 81 has an end provided with a protruding hook 86 for attaching elbow rest 80 to main body 10. To correspond to hook 86, main body 10 having a geometry shown for example in Fig. 14 (identical to that shown in Fig. 15) has a hook engagement hole 110 formed therein closer to a rear side lower than arm insertion portion 11. While this embodiment provides two hooks engagement holes 110, any number of holes 110 may be provided depending on the geometry of hook 86 and the number thereof.

Furthermore, main body 10 has a bottom surface 10a initially set to incline from a proximal side toward a distal side obliquely downward. As such, when main body 10 is placed for example on a table, inclined bottom surface 10a contacts the table, and main body 10 or arm insertion portion 11 thus inclines.

When electronic blood pressure monitor 1H has elbow rest 80 at an unused position, electronic blood pressure monitor 1H has elbow rest 80 removed from main body 10. When blood pressure is measured, elbow rest 80 has hook 86 inserted into hook engagement hole 110 of main body 10 to set elbow rest 80 at an elbow supporting position protruding from the rear side of main body 10. At this elbow supporting position, elbow rest 80 has distal support 82 with a bottom surface 82a in contact with the table and main body 10 is supported in an inclined position so that blood pressure can be measured with main body 10 improved in stability.

Although the present invention has been described and illustrated in detail, it is clearly understood that the same is by way of illustration and example only and is not to be taken by way of limitation, the spirit and scope of the present invention being limited only by the terms of the appended claims.

## Claims

1. An electronic blood pressure monitor having a main body (10) provided with an arm insertion portion (11) having a throughhole and provided with a cylindrical cuff, wherein said main body (10) is provided with an elbow rest (20, 30, 40, 50, 60, 70, 80) receiving an elbow of an arm inserted through and thus placed external to said arm insertion portion (11) and said elbow rest (20, 30, 40, 50, 60, 70, 80) is adapted to be positionally changeable between an elbow supporting position and an unused position.

2. The electronic blood pressure monitor according to claim 1, wherein said elbow rest (20, 30, 40, 50, 60, 80) is positionally changeable between said elbow supporting position and said unused position relative to said main body (10).

3. The electronic blood pressure monitor according to claim 2, wherein said elbow rest (20, 30, 40, 60) is supported between said elbow supporting position and said unused position rotatably by a support shaft (15) provided to said main body (10).

4. The electronic blood pressure monitor according to claim 3, wherein said elbow rest (20, 40) at said unused position is accommodated by said main body (10) upper than said support shaft (15).

5. The electronic blood pressure monitor according to claim 3, wherein said elbow rest (30, 60) at said unused position is accommodated by said main body (10) lower than said support shaft (15).

6. The electronic blood pressure monitor according to claim 3, wherein said elbow rest (60) at said unused position functions as a leg supporting said main body (10) and at said elbow supporting position functions as a leg supporting said main body (10) as well as an elbow rest.

7. The electronic blood pressure monitor according to claim 2, wherein said elbow rest (50) is slidably input to and output from and thus accommodated in said main body (10).

8. The electronic blood pressure monitor according to claim 2, wherein said elbow rest (80) is detachably attachable to said main body (10).

9. The electronic blood pressure monitor according to claim 1, wherein said elbow rest (70) is formed to be integral with said main body (10) to function as a leg supporting said main body (10) as well as an elbow rest when said elbow rest (70) has said elbow supporting position.

10. The electronic blood pressure monitor according to claim 9, wherein said elbow rest (70) protrudes from said main body (10).

11. The electronic blood pressure monitor according to claim 1, wherein said elbow rest (20, 40, 60, 70, 80) has a geometry supporting an inclined position of said main body (10) allowing said arm insertion portion (11) to be obliquely downward from a proximal side toward a distal side when said elbow rest (20, 40, 60, 70, 80) is positionally changed to take said elbow supporting position.

12. The electronic blood pressure monitor according to claim 1, wherein said elbow rest (20, 30, 40, 50, 60, 70, 80) includes a proximal support (21, 31, 41, 51, 61, 71, 81) supporting a proximal portion of an arm with respect to an elbow and a distal support (22, 32, 42, 52, 62, 72, 82) bent and thus directly adjacent to said proximal support (21, 31, 41, 51, 61, 71, 81) to support a distal portion of the arm with respect to the elbow, and said proximal support (21, 31, 41, 51, 61, 71, 81) and said distal support (22, 32, 42, 52, 62, 72, 82) form an inflection serving as an elbow support (23, 33, 43, 53, 63, 73, 83).
